# FASCICULE DE BREVET EUROPEEN

(11) **EP 0 850 906 B1**
(45) Date de publication et mention de la délivrance du brevet: **04.04.2001**
(21) Numéro de dépôt: 97403052.0
(22) Date de dépôt: 16.12.1997
(51) Int. Cl.: C07C 41/06, C07C 43/04

(54) **Procédé et dispositif d'étherification d'une charge oléfinique hydrocarbonée**
Verfahren und Vorrichtung zur Etherifizierung von olefinischen Kohlenwasserstoff-Einsätzen
Process and apparatus for the etherification of olefinic hydrocarbon feedstocks

(30) Priorité: 31.12.1996 FR 9616289
(43) Date de publication de la demande: 01.07.1998
(73) Titulaire: TOTAL RAFFINAGE DISTRIBUTION S.A., 92800 Puteaux (FR)
(72) Inventeur: Masson, Michel, 76170 Lillebonne (FR)
(74) Mandataire: Jolly, Jean-Pierre

(56) Documents cités:
- WO-A-96/21635
- US-A- 4 830 635

## Description

La présente invention concerne un procédé amélioré d'éthérification d'une charge oléfinique hydrocarbonée.

L'éthérification vise à obtenir, par réaction d'une charge oléfinique hydrocarbonée comprenant de 4 à 7 atomes de carbone avec un alcool comprenant de 1 à 3 atomes de carbone, des éthers à haut indice d'octane susceptibles de remplacer dans les essences les composés tels que les dérivés du plomb ou le benzène, qui sont nocifs et dont la teneur tend à être diminuée.

Les éthers les plus couramment produits sont:
- le MTBE (Méthyltertiobutyléther) à partir de l'isobutène et du méthanol;
- l'ETBE (Ethyltertiobutyléther) à partir de l'isobutène et de l'éthanol;
- le TAME (Tertioamylméthyléther) à partir de l'isoamylène et du méthanol;
- le TAEE (Tertioamyléthyléther) à partir de l'isoamylène et de l'éthanol.

Pour effectuer la réaction d'éthérification, la charge à traiter est mélangée à l'alcool selon des proportions massiques entre les isooléfines réactives et l'alcool variant de 1:1 à 5:1, puis dirigée vers au moins un réacteur contenant un catalyseur du type résine acide, en lit fixe ou bouillonnant. La température régnant dans ce réacteur est généralement comprise entre 20 et 100°C, et la pression entre 1-3 MPa (10 et 30 bars).

Les effluents sont ensuite pour partie recyclés à l'entrée du réacteur amont, et pour partie dirigés vers un fractionnement en fond duquel est soutirée une coupe éther dont la pureté est comprise entre 90 et 100%, et en tête duquel ressort la fraction de la charge à traiter n'ayant pas réagi. Cette dernière est alors admise au sein d'un second fractionnement, en fond duquel est retirée une coupe C4 purifiée, et en tête duquel ressort une coupe légère composée notamment des sous-produits de la réaction, qui est pour partie recyclée à l'entrée du réacteur amont.

La réaction d'éthérification étant fortement exothermique, les deux recycles précités vers le réacteur amont permettent de diluer la charge et donc de limiter la hausse de température au sein du réacteur, de manière à conduire la réaction le plus près possible de sa température optimale sur le plan de la thermodynamique.

Toutefois, ces recycles présentent certains inconvénients. Ils nécessitent tout d'abord la mise en place d'équipements supplémentaires, telles que des pompes ou des échangeurs, ainsi que des fractionnements de taille plus importante, du fait du débit supérieur de fluide qui y circule. Ils génèrent en outre des consommations supplémentaires, notamment en électricité et en eau de refroidissement.

De plus, étant donné que l'on recycle vers l'entrée du réacteur une partie des effluents de celui-ci, et donc en particulier de l'éther, la réaction équilibrée [Alcool + Charge ⇐ ⇒ Ether] est déplacée vers la gauche comme le montrent les lois de Le Chatelier, ce qui est pénalisant pour la production d'éther

Le brevet américain n° US 4 830 635 concerne un procédé d'éthérification d'une charge oléfinique hydrocarbonée, comprenant le recyclage au réacteur d'une coupe d'essence C5-C9, issue d'un procédé de conversion d'oléfines (procédé "MOGD"); cette coupe d'essence est riche en oléfines (cf. colonne 6, lignes 19-51) et peut contenir au moins 85% d'iso-oléfines en C5 et C6 (cf. colonne 8, lignes 55-60). Un tel procédé conduit à une production élevée de sous-produits réactionnels indésirables, générant une exothermicité supplémentaire; en outre, l'activité du catalyseur d'éthérification diminue rapidement.

Afin de remédier aux différents inconvénients évoqués ci-dessus, la présente invention vise un procédé d'éthérification permettant d'obtenir une meilleure conversion de la charge en éther, en mettant en oeuvre un appareillage moins coûteux que celui qui est utilisé dans l'art antérieur.

A cet effet, la présente invention a pour objet un procédé d'éthérification d'une charge oléfinique hydrocarbonée comprenant de 4 à 7 atomes de carbone, par un alcool comprenant de 1 à 3 atomes de carbone, dans lequel la charge à traiter passe, à une pression de 1-3 MPa (10 à 30 bars) et à une température de 20 à 100°C, dans au moins un réacteur chargé avec un catalyseur d d'éthérification, une coupe d'hydrocarbures dont le point d'ébullition est compris entre celui de la charge et 200°C étant introduite en amont dudit réacteur et en appoint de la charge.

Ce procédé se caractérise en ce que la coupe d'appoint a une teneur en isooléfines inférieure à 10%.

Le procédé conforme à l'invention permet d'atteindre les objectifs précités. En effet, l'injection d'une coupe d'hydrocarbures en appoint de la charge permet de remplacer les recycles prévus dans l'art antérieur, de sorte que les installations nombreuses et coûteuses qui sont nécessaires à ces recycles sont supprimées au profit d'une simple conduite d'arrivée de cette coupe d'appoint.

Cette coupe d'appoint permet en outre une parfaite dilution de la charge, et améliore le rendement en éther puisqu'elle est parfaitement neutre vis-à-vis de la réaction d'éthérification, alors que le recycle des effluents réactionnels tel que pratiqué dans l'art antérieur apparaît préjudiciable à cette réaction.

Enfin, l'injection de cette coupe d'appoint ne perturbe nullement le fonctionnement de l'unité d'éthérification, et permet au contraire de mieux l'intégrer au sein de l'ensemble des unités d'une raffinerie. En effet, cette coupe possède un point d'ébullition supérieur à celui de la charge. Elle se trouvera donc entièrement soutirée en fond du fractionnement postérieur à la zone réactionnelle et ne sera pas mélangée à la fraction de la charge à traiter n'ayant pas réagi.

De plus, le point d'ébullition de cette coupe d'appoint est inférieur à 200°C, ce qui correspond à la limite haute des essences. De la sorte, le mélange d'éther et de coupe d'appoint recueilli en fond du fractionnement postérieur à la zone réactionnelle, est une essence à fort indice d'octane (du fait de la présence de l'éther) et peut donc être valorisé sans traitement ni séparation complémentaire.

De manière avantageuse, la coupe d'appoint a une teneur en isooléfines inférieure à 5%, car les oléfines ont un effet négatif sur le catalyseur d'éthérification, et conduisent à une production élevée de sous-produits réactionnels indésirables, générant une exothermicité supplémentaire.

Selon une caractéristique avantageuse de l'invention, le rapport massique entre la coupe d'appoint et le mélange charge/alcool, est compris entre 0,1 et 10. En effet, le débit de coupe d'appoint doit être d'une part suffisant pour permettre une dilution de la charge et donc compenser l'exothermicité de la réaction, et d'autre part pas trop élevé pour des raisons de dimensionnement des différents appareillages de l'unité.

De préférence, la coupe d'appoint est introduite à une température de 20 à 100°C, et sous une pression de 1-3 MPa (10 à 30 bars), de sorte que le mélange entre la charge, l'alcool et cette coupe d'appoint soit admis au sein du réacteur d'éthérification dans les conditions classiques de température et de pression.

De manière avantageuse, la coupe d'appoint possède un indice d'octane recherche supérieur à 80, et de préférence à 90. Ainsi, le soutirat recueilli en fond du fractionnement postérieur à la zone réactionnelle, qui est composé d'éther et de coupe d'appoint, possède un indice d'octane encore plus élevé, qui peut être supérieur à 100. Ce soutirat est donc valorisable comme produit de base pour essence de très bonne qualité, et susceptible d'être utilisé soit pur, soit en mélange avec d'autres produits de base pour essence à plus faible indice d'octane.

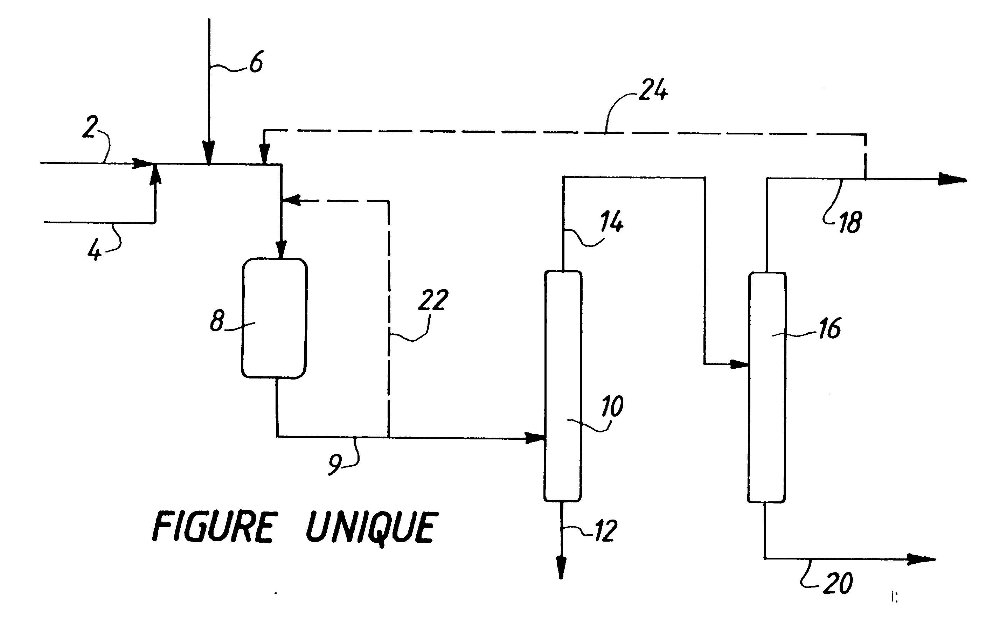

Selon une caractéristique préférée de l'invention, la coupe d'appoint possède un point d'ébullition supérieur d'au moins 20°C, et de préférence d'au moins 30°C, à celui de la charge. Ceci permet une meilleure séparation entre la coupe d'appoint et la charge au niveau du fractionnement postérieur à la zone réactionnelle. D'autres caractéristiques et avantages de l'invention apparaissent à la lecture de la description d'un mode particulier de réalisation, faite ci-après en référence à la figure unique qui est une vue schématique d'une unité d'éthérification incluant l'arrivée d'une coupe d'appoint conforme à l'invention.

La présente description va uniquement faire référence à la fabrication de MTBE (Méthyltertiobutyléther) à partir d'isobutène et de méthanol. Néanmoins, l'invention trouve son application dans la fabrication de tout éther à partir d'une charge hydrocarbonée comprenant de 4 à 7 atomes de carbone, et d'un alcool comprenant de 1 à 3 atomes de carbone.

Comme le montre cette figure unique, l'isobutène réactif arrive par la ligne 2 et se trouve tout d'abord mélangée à du méthanol qui circule dans la ligne 4. Le rapport molaire entre ces deux constituants est voisin des proportions stoechiométriques de la réaction, et se trouve par exemple égal à 1 : 1.

L'isobutène et le méthanol sont ensuite mélangés à une coupe d'appoint arrivant par la ligne 6, dont la nature et la fonction seront explicitées par la suite. Ce mélange ternaire est alors admis au sein d'un réacteur d'éthérification 8 chargé avec une résine de type acide. Les conditions opératoires qui y règnent sont par exemple une température d'entrée de 50°C et une pression de 20 bars. L'unité représentée sur la figure inclut un réacteur unique, mais elle pourrait naturellement comprendre au moins un réacteur supplémentaire.

Les effluents de la zone réactionnelle (en l'occurence, sur la figure, les effluents du réacteur 8) sont dirigés par une ligne 9, vers un fractionnement 10 en fond duquel est soutirée, par la ligne 12, une coupe riche en MTBE, et en tête duquel ressort, par la ligne 14, la fraction de la charge à traiter n'ayant pas réagi. Cette dernière est alors admise au sein d'un second fractionnement 16, en fond duquel est retirée, par la ligne 20, une coupe C4 purifiée, et en tête duquel ressort, par la ligne 18, une coupe légère composée notamment des sous-produits de la réaction d'éthérification.

La nature et la fonction de la coupe d'appoint arrivant par la ligne 6 vont maintenant être explicitées. Cette coupe permet une dilution de la charge avant son admission dans le réacteur 8, et limite donc l'augmentation de température au sein de celui-ci due à l'exothermicité de la réaction, permettant à celle-ci de se dérouler dans des conditions proches de l'optimum thermodynamique.

Cette coupe d'appoint est par exemple une essence dont le point d'ébullition est supérieur d'environ 30°C à celui de la charge. De la sorte, elle est entièrement soutirée en fond du fractionnement 10 et ne se trouve pas mélangée à la fraction de la charge à traiter n'ayant pas réagi, évacuée par la ligne 14. La coupe d'appoint-MTBE soutirée par la ligne 12 constitue alors un produit de base pour essence à fort indice d'octane, qui est directement valorisable sans traitement ni séparation supplémentaire.

L'injection de cette coupe d'appoint permet de supprimer les recycles de l'art antérieur représentés en pointillés, constitués pour partie par les effluents réactionnels (ligne 22), et pour partie par la fraction de tête du second fractionnement 16 (ligne 24). Ceci se traduit donc par une réduction substantielle de l'investissement nécessaire à l'unité.

L'utilisation comme diluant de cette coupe d'appoint améliore en outre le rendement en MTBE par rapport à ce que l'on obtient en recyclant les effluents réactionnels par la ligne 22. En effet, si l'on admet en charge du réacteur ce recycle riche en MTBE, on contribue à déplacer la réaction équilibrée d'éthérification dans le sens d'une moindre production d'éther, comme l'enseignent les lois de Le Chatelier, alors que la coupe d'appoint est parfaitement neutre vis-à-vis de cette réaction.

On notera que, sur la figure annexée, l'isobutène est tout d'abord mélangé au méthanol puis à la coupe d'appoint. Il va de soi que ce mélange ternaire peut être obtenu dans un ordre quelconque.

L'exemple ci-après, qui n'a pas de caractère limitatif, est destiné à illustrer la mise en oeuvre de l'invention et les avantages de celle-ci.

### EXEMPLE COMPARATIF

Une coupe C4, constituée notamment de 22.5% en poids d'isobutylène, de 21% d'isoparaffines et de 8.6% de n-paraffines, bouillant à -5°C et dont le débit est de 20 t/h, ainsi que du méthanol, dont le débit est de 2.9 t/h, sont envoyés en charge d'un réacteur d'éthérification.

Ce réacteur fonctionne sous une pression de 1.9 MPa (19 bars), une température d'entrée de 60°C et une vitesse volumique horaire (VVH) de 0.33/h (il s'agit du rapport du débit horaire de la charge en m³/h sur le volume de catalyseur en m³). Il est chargé avec un catalyseur du type résines acides.

Dans un premier temps, on procède selon l'art antérieur : on effectue vers l'entrée du réacteur un recycle de MTBE (Méthyltertiobutyléther) à raison de 8,2 t/h.

L'effluent du réacteur est dirigé vers un débutaniseur, en tête duquel on trouve la fraction de la coupe C4 n'ayant pas réagi, et en fond duquel on soutire une coupe riche en MTBE. La conversion de l'isobutylène en MTBE est alors d'environ 87%.

Dans un second temps, on procède selon l'invention : la même coupe C4 que précédemment, ainsi que du méthanol, les deux ayant respectivement les mêmes débits que ci-dessus, sont mélangés à une essence d'appoint constituée d'isooctane, bouillant à 120°C, et dont le débit est de 11 t/h. Ce mélange ternaire ainsi constitué est ensuite envoyé vers un réacteur d'éthérification fonctionnant dans les mêmes conditions opératoires que celles décrites ci-dessus.

L'effluent du réacteur est dirigé vers un débutaniseur, en tête duquel on trouve la fraction de la coupe C4 n'ayant pas réagi, et en fond duquel on soutire une coupe mixte d'isooctane et de MTBE. La conversion de l'isobutylène en MTBE a augmenté pour atteindre environ 96%.

Ainsi, comme on peut le constater, le procédé conforme à l'invention permet de supprimer le recycle des effluents du réacteur ce qui, outre des avantages économiques liés à la réduction de l'investissement, augmente la conversion de l'isobutylène en MTBE.

## Revendications

1. Procédé d'éthérification d'une charge oléfinique hydrocarbonée comprenant de 4 à 7 atomes de carbone, par un alcool comprenant de 1 à 3 atomes de carbone, dans lequel la charge à traiter passe, à une pression de 10 à 30 bars et à une température de 20 à 100°C, dans au moins un réacteur chargé avec un catalyseur d'éthérification, une coupe d'hydrocarbures dont le point d'ébullition est compris entre celui de la charge et 200°C étant introduite en amont dudit réacteur et en appoint de la charge, **caractérisé en ce que** la coupe d'appoint a une teneur en isooléfines inférieure à 10%.

2. Procédé selon la revendication précédente, caractérisé en ce que la coupe d'appoint a une teneur en isooléfines inférieure à 5%.

3. Procédé selon l'une des revendications précédentes, caractérisé en ce que le rapport massique entre la coupe d'appoint et le mélange charge/alcool, est compris entre 0,1 et 10.

4. Procédé selon l'une des revendications précédentes, caractérisé en ce que la coupe d'appoint est introduite à une température de 20 à 100°C, et sous une pression de 1-3 MPa (10 à 30 bars).

5. Procédé selon l'une des revendications précédentes, caractérisé en ce que la coupe d'appoint possède un indice d'octane recherche supérieur à 80.

6. Procédé selon la revendication précédente, caractérisé en ce que la coupe d'appoint possède un indice d'octane recherche supérieur à 90.

7. Procédé selon l'une des revendications précédentes, caractérisé en ce que la coupe d'appoint possède un point d'ébullition supérieur d'au moins 20°C à celui de la charge.

8. Procédé selon la revendication précédente, caractérisé en ce que la coupe d'appoint possède un point d'ébullition supérieur d'au moins 30°C à celui de la charge.

## Claims

1. Method of etherifying a hydrocarbonated olefinic charge, having 4 to 7 carbon atoms, by means of an alcohol having 1 to 3 carbon atoms, wherein the charge to be treated passes, at a pressure of 10 to 30 bars and at a temperature of 20 to 100° C, into at least one reactor charged with an etherification catalyst, a quantity of hydrocarbons with a boiling point of between that of the charge and 200° C being introduced upstream of said reactor and in addition to the charge, characterised in that the additive has an isoolefin content of less than 10 %.

2. Method according to the preceding claim, characterised in that the additive has an isoolefin content of less than 5 %.

3. Method according to one of the preceding claims, characterised in that the mass ratio between the additive and the mixture of charge and alcohol is between 0.1 and 10.

4. Method according to one of the preceding claims, characterised in that the additive is introduced at a temperature of between 20 and 100° C and at a pressure of 1 - 3 MPa (1 to 30 bars).

5. Method according to one of the preceding claims, characterised in that the additive has a specific octane number greater than 80.

6. Method according to the preceding claim, characterised in that the additive has a specific octane number greater than 90.

7. Method according to one of the preceding claims, characterised in that the additive has a boiling point higher by at least 20° C than that of the charge.

8. Method according to the preceding claim, characterised in that the additive has a boiling point higher by at least 30° C than that of the charge.

## Patentansprüche

1. Verfahren zur Etherifizierung von olefinischen Kohlenwasserstoff-Einsätzen mit vier bis sieben Kohlenstoffatomen mit einem, einem bis drei Kohlenstoffatome aufweisenden Alkohol, in den der genannte Einsatz unter einem Druck von 10 bar bis 30 bar und bei einer Temperatur von 20° C bis 100° C eingeführt wird, in wenigstens einem, mit einem Etherifizierungskatalysator ausgerüsteten Reaktor, wobei stromaufwärts bezüglich des Reaktors und zusätzlich zu dem genannten Einsatz eine Teilmenge an Kohlenwasserstoffen zugegeben wird, deren Siedepunkt zwischen demjenigen des Einsatzes und 200° C liegt, dadurch gekennzeichnet, dass die zusätzliche Teilmenge einen Gehalt an Isoolefinen von weniger als 10% aufweist.

2. Verfahren gemäß dem vorangegangenen Anspruch, dadurch gekennzeichnet, dass die zusätzliche Teilmenge einen Gehalt an Isoolefinen von weniger als 5% aufweist.

3. Verfahren nach einem der vorangegangenen Ansprüche, dadurch gekennzeichnet, dass das Massenverhältnis zwischen der zusätzlichen Teilmenge einerseits und der aus dem genannten Einsatz und dem Alkohol bestehenden Mischung andererseits zwischen 0,1 und 10 liegt.

4. Verfahren nach einem der vorangegangenen Ansprüche, dadurch gekennzeichnet, dass die zusätzliche Teilmenge bei einer Temperatur von 20° C bis 100° C und unter einem Druck von 1 MPa bis 3 MPa (10 bar bis 30 bar) eingeführt wird.

5. Verfahren nach einem der vorangegangenen Ansprüche, dadurch gekennzeichnet, dass die zusätzliche Teilmenge eine Researchoktanzahl (R.O.Z.) von mehr als 80 aufweist.

6. Verfahren gemäß dem vorangegangenen Anspruch, dadurch gekennzeichnet, dass die zusätzliche Teilmenge eine Researchoktanzahl (R.O.Z.) von mehr als 90 aufweist.

7. Verfahren nach einem der vorangegangenen Ansprüche, dadurch gekennzeichnet, dass die zusätzliche Teilmenge einen Siedepunkt aufweist, der wenigstens 20° C über demjenigen des Einsatzes liegt.

8. Verfahren gemäß dem vorangegangenen Anspruch, dadurch gekennzeichnet, dass die zusätzliche Teilmenge einen Siedepunkt aufweist, der wenigstens 30° C über demjenigen des Einsatzes liegt.
